# EUROPEAN PATENT APPLICATION

(11) **EP 1 579 867 A1**
(43) Date of publication of application: **28.09.2005**
(21) Application number: 03778813.0
(22) Date of filing: 11.12.2003
(51) Int. Cl.: A61K 38/17, A61P 9/00, A61P 9/04

(54) **MEDICINAL COMPOSITION FOR TREATING ISCHEMIC CARDIAC FAILURE**

(30) Priority: 13.12.2002 JP 2002362511
(71) Applicant: Fujiwara, Hisayoshi, Gifu 502-0071 (JP)
(72) Inventor: FUJIWARA, Hisayoshi, Gifu-shi, Gifu 502-0071 (JP); TAKEMURA, Genzou, Gifu-shi, Gifu 502-0936 (JP)
(74) Representative: Vossius & Partner
(86) International application number: PCT/JP2003/015870
(87) International publication number: WO 2004/054604

(57) **Abstract**

The object of the present invention is to provide a pharmaceutical composition for treating non-ischemic heart failure caused by exacerbation of cardiomyopathy. The long-term administration of a colony-stimulating factor ameliorates progressive myocardial fibrosis, left ventricular remodeling and heart failure.

## Description

### TECHNICAL FIELD

The present invention relates to a pharmaceutical composition for treating non-ischemic heart failure, which comprises a colony-stimulating factor as an active ingredient. The present invention also relates to a method for treating non-ischemic heart failure, which comprises administering a colony-stimulating factor.

### BACKGROUND ART

Heart failure is a condition in which decreased cardiac function makes it impossible to ensure blood ejection required for tissue metabolism throughout the body, or a condition in which such blood ejection is achieved only upon elevation of ventricular filling pressure [Shin Rinsho Naikagaku (New Clinical Internal Medicine), Igaku-Shoin Ltd., Japan]. Ischemic heart failure is hypoxia-induced heart failure caused by failing to receive the blood volume required for metabolism, while non-ischemic heart failure refers to heart failure other than ischemic heart failure. Non-ischemic heart failure encompasses that caused by exacerbation of cardiomyopathy such as idiopathic, secondary or other forms of cardiomyopathy.

Human granulocyte colony-stimulating factor (G-CSF) is a hematopoietic factor found as a differentiation and proliferation factor for hematopoietic progenitor cells of granulocytic lineage and is clinically used as a therapeutic agent for neutropenia following bone marrow transplantation or cancer chemotherapy because it promotes *in vivo* hematopoiesis of neutrophils. In addition to the above effect, human G-CSF acts on hematopoietic stem cells to stimulate their proliferation and differentiation. Moreover, the inventors of the present invention have recently reported that G-CSF will induce recruitment of hematopoietic stem cells from the bone marrow into the peripheral blood and will promote their migration to myocardial infarction lesions and their differentiation into cardiac muscle cells, thereby alleviating left ventricular remodeling and heart failure following myocardial infarction (Minatoguchi, S. et al. Circulation, 2004 (in press)).

However, it has been unclear whether G-CSF is also effective for non-ischemic heart failure caused by cardiomyopathy such as dilated cardiomyopathy.

### DISCLOSURE OF THE INVENTION

The object of the present invention is to provide a pharmaceutical composition for treating non-ischemic heart failure caused by exacerbation of cardiomyopathy.

The inventors of the present invention have found that the long-term administration of G-CSF ameliorates progressive myocardial fibrosis, left ventricular remodeling and heart failure in an animal model of cardiomyopathy. This finding led to the completion of the present invention.

Namely, the present invention provides a pharmaceutical composition for treating non-ischemic heart failure, which comprises a colony-stimulating factor as an active ingredient.

The present invention also provides a method for treating non-ischemic heart failure, which comprises administering to a patient in need thereof a colony-stimulating factor as an active ingredient in an amount effective for treating non-ischemic heart failure.

The present invention further relates to the use of a colony-stimulating factor for the manufacture of a pharmaceutical composition for treating non-ischemic heart failure.

The present invention further provides a kit for treating non-ischemic heart failure, which comprises a colony-stimulating factor in an amount effective for treating non-ischemic heart failure, and instructions for use.

### BRIEF DESCRIPTION OF DRAWINGS

Figure 1 shows photomicrographs of ventricular cross-section specimens stained by Masson's trichrome stain. (A) Control group, (B) G-CSF group. Areas stained in blue are fibrotic areas (indicated with arrows).
Figure 2 shows photomicrographs of bone marrow cell-derived cardiac muscle cells observed under a confocal laser scanning microscope. (A) DiI-labeled bone marrow cells (red, thin arrows), (B) Nuclear staining (blue, bold arrows), (C) Cardiac muscle cells stained with α-sarcomeric actin (green), (D) Merged image of (A) to (C). Scale bar is 20 µm.

### BEST MODE FOR CARRYING OUT THE INVENTION

The present invention relates to a pharmaceutical composition for treating non-ischemic heart failure, which comprises a colony-stimulating factor as an active ingredient. Non-ischemic heart failure encompasses, for example, that caused by exacerbation of cardiomyopathy. Examples of cardiomyopathy include idiopathic cardiomyopathy such as dilated, hypertrophic and restrictive forms of cardiomyopathy, as well as secondary cardiomyopathy such as myocarditis and sarcoidosis. The present invention can be used for dilated cardiomyopathy, by way of example.

Examples of a colony-stimulating factor include granulocyte colony-stimulating factor (G-CSF), granulocyte-monocyte colony-stimulating factor (GM-CSF), and monocyte colony-stimulating factor (M-CSF). For example, G-CSF can be used in the present invention.

In a case where G-CSF is used as an active ingredient in the pharmaceutical composition of the present invention, any G-CSF can be used, but a highly purified one is preferred. Specific examples include mammalian G-CSF, particularly human G-CSF or an equivalent thereof having substantially the same biological activities. G-CSF used herein may be of any origin, including those naturally occurring and those produced recombinantly. Such recombinantly produced G-CSF may have the same amino acid sequence as naturally-occurring G-CSF (e.g., JP 2-5395 B, JP 62-236488 A) or may comprise deletion, substitution and/or addition of one or more amino acid residues in the amino acid sequence as long as it retains the same biological activities as naturally-occurring G-CSF. For example, site-specific mutagenesis (Gotoh, T. et al. (1995) Gene 152, 271-275; Zoller, M.J. And Smith, M. (1983) Methods Enzymol. 100, 468-500; Kramer, W. et al. (1984) Nucleic Acids Res. 12, 9441-9456; Kramer, W. and Fritz H.J. (1987) Methods Enzymol. 154, 350-367; Kunkel, T.A. (1985) Proc. Natl. Acad. Sci. USA, 82, 488-492; Kunkel (1988) Methods Enzymol. 85, 2763-2766) or other techniques can be used to introduce appropriate mutations into the amino acid sequence of G-CSF, to thereby prepare a polypeptide functionally equivalent to G-CSF. Likewise, amino acid mutations may also occur in the natural world. It is already known that a polypeptide having an amino acid sequence modified from another amino acid sequence by deletion and/or addition of one or more amino acid residues and/or by their substitution with other amino acids retains the same biological activities as the original polypeptide (Mark, D.F. et al., Proc. Natl. Acad. Sci. USA (1984) 81, 5662-5666; Zoller, M.L. & Smith, M. Nucleic Acids Research (1982) 10, 6487-6500; Wang, A. et al., Science (1984) 224, 1431-1433; Dalbadie-McFarland, G. et al., Proc. Nat 1. Acad. Sci. USA (1982) 79, 6409-6413).

Thus, a polypeptide which has an amino acid sequence comprising one or more amino acid mutations in the amino acid sequence of G-CSF and which is functionally equivalent to G-CSF can also be used in the pharmaceutical composition of the present invention. The number of mutated amino acids in such a polypeptide is usually up to 30 amino acids, preferably up to 15 amino acids, and more preferably up to 5 amino acids (e.g., up to 3 amino acids).

In substitution mutants, amino acid substitutions desirably occur in such a manner as to conserve the nature of amino acid side chains. Examples of amino acids available for such amino acid substitutions include hydrophobic amino acids (A, I, L, M, F, P, W, Y, V), hydrophilic amino acids (R, D, N, C, E, Q, G, H, K, S, T), amino acids having an aliphatic side chain (G, A, V, L, I, P), amino acids having a hydroxy-containing side chain (S, T, Y), amino acids having a sulfur-containing side chain (C, M), amino acids having a carboxylic acid- or amide-containing side chain (D, N, E, Q), amino acids having a base-containing side chain (R, K, H), and amino acids having an aromatic-containing side chain (H, F, Y, W) (capital letters in parentheses refer to the corresponding amino acids in single-letter notation).

Polypeptides comprising addition of several amino acid residues in the amino acid sequence of G-CSF encompass fusion polypeptides containing G-CSF. Fusion polypeptides are fusion products between G-CSF and other polypeptides, and such polypeptides can also be used in the present invention. To prepare a fusion polypeptide, for example, DNA encoding G-CSF and DNA encoding a different polypeptide may be ligated together in-frame and introduced into a suitable expression vector, followed by expression in a suitable host. Other polypeptides to be fused with G-CSF are not limited in any way as long as the resulting fusion polypeptides retain the same biological activities as G-CSF.

There are already many reports of G-CSF derivatives having mutations in the amino acid sequence of G-CSF; it is possible to use these known G-CSF derivatives (e.g., USP 5,581,476, USP 5,214,132, USP 5,362,853, USP 4,904,584).

Moreover, it is also possible to use chemically modified G-CSF. Examples of chemically modified G-CSF include those modified by structural alteration, addition and/or deletion of sugar chains, as well as those conjugated with a compound such as polyethylene glycol (e.g., USP 5,824,778, USP 5,824,784, WO96/11953, WO95/21629, WO94/20069, USP 5,218,092, JP 4-164098 A).

G-CSF used in the present invention may be prepared in any manner; for example, by culturing human tumor cell lines or by genetically engineered production in *E*. *coli* cells; yeast cells; mammalian cells such as Chinese hamster ovary (CHO) cells, C127 cells, COS cells, myeloma cells or BHK cells; or insect cells. G-CSF thus prepared is extracted, isolated and purified in various manners before use (e.g., JP 1-44200 B, JP 2-5395 B, JP 62-129298 A, JP 62-132899 A, JP 62-236488 A, JP 64-85098 A).

If necessary, depending on the administration mode and the dosage form, the pharmaceutical composition of the present invention for treating non-ischemic heart failure may be supplemented as appropriate with a suspending agent, a solubilizing agent, a stabilizing agent, an isotonizing agent, a preservative, an anti-adsorption agent, a surfactant, a diluent, an excipient, a pH adjustor, a soothing agent, a buffering agent, a sulfur-containing reducing agent, an antioxidant or the like.

Examples of a suspending agent include methylcellulose, Polysorbate 80, hydroxyethylcellulose, gum arabic, powdered tragacanth, carboxymethylcellulose sodium, and polyoxyethylenesorbitan monolaurate.

Examples of a solubilizing agent include polyoxyethylene hydrogenated castor oil, Polysorbate 80, nicotinamide, polyoxyethylenesorbitan monolaurate, Magrogol, and an ethyl ester of castor oil fatty acid.

Examples of a stabilizing agent include Dextran 40, methylcellulose, gelatin, sodium sulfite, and sodium metasulfite.

Examples of an isotonizing agent include D-mannitol and sorbitol.

Examples of a preservative include methyl parahydroxybenzoate, ethyl parahydroxybenzoate, sorbic acid, phenol, cresol, and chlorocresol.

Examples of an anti-adsorption agent include human serum albumin, lecithin, dextran, ethylene oxide-propylene oxide copolymers, hydroxypropylcellulose, methylcellulose, polyoxyethylene hydrogenated castor oil, and polyethylene glycol.

Examples of a sulfur-containing reducing agent include those having a sulfhydryl group such as N-acetylcysteine, N-acetylhomocysteine, thioctic acid, thiodiglycol, thioethanolamine, thioglycerol, thiosorbitol, thioglycolic acid and a salt thereof, sodium thiosulfate, glutathione, and a C₁-C₇ thioalkanoic acid.

Examples of an antioxidant include erythorbic acid, dibutylhydroxytoluene, butylhydroxyanisole, α-tocopherol, tocopherol acetate, L-ascorbic acid and a salt thereof, L-ascorbyl palmitate, L-ascorbyl stearate, sodium bisulfite, sodium sulfite, triamyl gallate and propyl gallate, as well as chelating agents such as ethylenediaminetetraacetic acid disodium salt (EDTA), sodium pyrophosphate and sodium metaphosphate.

Furthermore, the pharmaceutical composition of the present invention may comprise other commonly used ingredients, e.g., inorganic salts such as sodium chloride, potassium chloride, calcium chloride, sodium phosphate, potassium phosphate and sodium bicarbonate, as well as organic salts such as sodium citrate, potassium citrate and sodium acetate.

The pharmaceutical composition of the present invention for treating non-ischemic heart failure may be administered in, but is not limited to, a dosage form of injections (e.g., subcutaneous, intracutaneous, intramuscular, intravenous or intraperitoneal injections), in any dosage form suitable for transdermal, transmucosal or transnasal administration or in any dosage form suitable for oral administration (e.g., tablets, capsules, granules, solutions, suspensions).

In the pharmaceutical composition of the present invention, the dose and frequency of its administration can be determined as appropriate by those skilled in the art by taking into account symptoms of a diseased patient to be treated. Without being limited thereto, the single dose of G-CSF is usually 0.1 to 100 µg/kg per adult, preferably 1 to 10 µg/kg per adult, and usually administered for 1 to 7 days per week on a once- to three-times-α-day basis.

The pharmaceutical composition of the present invention for treating non-ischemic heart failure, which comprises a colony-stimulating factor as an active ingredient, is effective for treatment and/or prevention of non-ischemic heart failure.

The kit of the present invention comprises a colony-stimulating factor, one or more pharmaceutically acceptable and appropriate carriers or the like (if desired), as well as instructions for use.

The present invention will now be further described in the following examples, which are not intended to limit the scope of the present invention.

### EXAMPLES

An animal model of cardiomyopathy was used to examine whether G-CSF administration would prevent the progression of cardiomyopathy-induced heart failure.

### (1) Experimental procedures

### Animal model of heart failure:

UM-X7.1 cardiomyopathic hamsters are deficient in the δ-sarcoglycan gene (Sakamoto A. et al., Proc. Natl. Acad. Sci. USA 1997, 94: 13873-13878) and begin to lose cardiac muscle cells at 4 weeks of age after birth (Jasmin G. et al., Muscle Nerve 1982, 5: 20-25). Subsequently, the animals undergo progressive formation of multiple lesions with loss of cardiac muscle cells and develop significant fibrosis, which causes auxocardia and/or hypodynamia cordis and eventually leads to heart failure death. In the natural course, the survival rate at 30 weeks of age is about 50%.

### Procedures for G-CSF administration:

Experiment 1: Sixteen male UM-X7.1 cardiomyopathic hamsters (15 weeks of age after birth) were treated with an administration schedule in which 10 µg/kg/day G-CSF was subcutaneously injected for 5 consecutive days, followed by withdrawal for 2 days. This treatment was continued for 15 weeks until 30 weeks of age. As a control group, 15 male UM-X7.1 hamsters of the same age were also subcutaneously injected with distilled water in the same manner. These two groups were compared for survival rate and evaluated hemodynamically, histologically and biochemically at 30 weeks of age.

Experiment 2: After 2 week administration of G-CSF or distilled water (n=6 in each group) according to the above administration schedule, the animals were intraperitoneally injected with Evans blue dye to examine uptake of this dye into cardiac muscle cells.

Experiment 3: The following experiment was further conducted to examine the presence or absence of recruitment of bone marrow cells to cardiac muscle and their differentiation into cardiac muscle cells. Bone marrow cells were collected from the femur of each male UM-X7.1 hamster (15 weeks of age after birth), labeled with DiI dye and then replanted into the medullary cavity of the animal. After administration of G-CSF or distilled water (n=6 in each group) according to the above administration schedule, cardiac muscle from each animal was examined histologically after 2 weeks.

### Hemodynamic evaluation:

This evaluation was accomplished by echocardiography using ultrasonic diagnostic equipment SSD-2000 (Aloka).

### Histological evaluation:

In Experiment 1, 10% formalin-fixed and paraffin-embedded ventricular cross sections (4 µm) were stained by Masson's trichrome stain to identify myocardial fibrotic areas, followed by quantitative analysis using an image analyzer Luzex-F (NIRECO). Also, another part of each heart was subjected to electron microscopic fixation and observed under an electron microscope.

Hearts from Experiments 2 and 3 were prepared into frozen specimens.

### Biochemical evaluation:

Gelatin-zymography was used to examine the activity of matrix metalloproteases (MMP) in cardiac muscle tissue.

### Confocal laser scanning microscope:

Frozen sections (6 µm) of cardiac muscle prepared from the animals receiving replantation of DiI-labeled bone marrow cells were immunofluorescently stained with α-sarcomeric actin and then observed under a confocal laser scanning microscope (Zweiss).

### Immunohistochemistry:

Paraffin sections (4 µm) of cardiac muscle tissue were immunostained with anti-δ-sarcoglycan antibody using an ABC kit (Vector Laboratories).

### Statistical analysis:

Numerical data were expressed as mean ± standard error. Comparison between two groups was made by Student's t-test and comparison of survival rate was made by Kaplan-Meier method. p<0.05 was considered as statistically significant.

### (2) Results

### Survival rate:

The survival rate at 30 weeks of age was 100% in the G-CSF group and 53% in the control group, indicating that G-CSF administration provided a significant improvement in survival rate (p<0.0001).

### Hemodynamics and left ventricular remodeling:

The echocardiographic evaluation demonstrated that the G-CSF group showed improved left ventricular systolic function because there were significant improvements in both left ventricular ejection fraction (EF) and left ventricular fraction shortening (%FS) of the G-CSF group. Moreover, the evaluation also demonstrated prevention of left ventricular remodeling in the G-CSF group because the G-CSF group showed significant reductions in both left ventricular end-diastolic diameter (LVEDD) and left ventricular end-systolic diameter (LVESD) as compared to the control group (Table 1).

When organ weights were measured at autopsy, the ratios of heart/body weight and lung/body weight were significantly smaller in the G-CSF group than in the control group, although there was no difference in body weight between these two groups (Table 1).

**Table 1**

| Comparison of parameters in hemodynamics and left ventricular remodeling | | | |
|---|---|---|---|
| | Control group | G-CSF group | p value |
| EF (%) | 29.4 ± 10.6 | 42.6 ± 9.2 | <0.0001 |
| %FS (%) | 12.0 ± 4.8 | 18.2 ± 4.7 | 0.0001 |
| LVEDD (mm) | 7.7 ± 1.1 | 6.8 ± 0.8 | 0.0038 |
| LVESD (mm) | 6.8 ± 1.2 | 5.6 ± 0.8 | 0.0001 |
| Body weight (g) | 130 ± 16 | 132 ± 11 | |
| Heart/body weight ratio | 0.4 1 ± 0.04 | 0.36 ± 0.05 | 0.0134 |
| Lung/body weight ratio | 0.82 ± 0.35 | 0.56 ± 0.10 | 0.0106 |

### Left ventricular fibrosis:

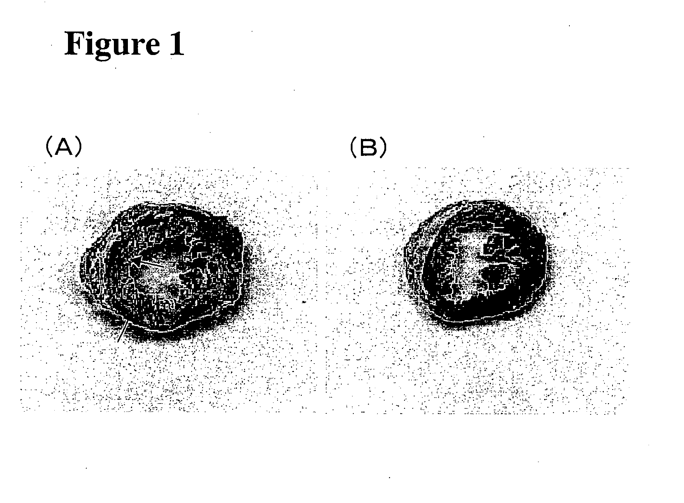

The percent area of left ventricular fibrosis was significantly reduced in the G-CSF group (8.6 ± 4.0%) as compared to the control group (20.2 ± 6.5%) (p<0.0001) (Figure 1).

Gelatin-zymography demonstrated that the G-CSF group showed increased activities of MMP-2 and MMP-9 in cardiac muscle tissue as compared to the control group (data not shown).

### Ultramicromorphology of cardiac muscle cells:

The 30-week-old hamsters in the control group were found to have, in their hearts, a large number of cardiac muscle cells containing degraded mitochondria and showing autophagic degeneration, whereas the G-CSF group had a reduced frequency of such cells.

### Cell membrane permeability (fragility) of cardiac muscle cells:

In Experiment 2, the control group was found to have a large number of cardiac muscle cells which took up Evans blue dye, i.e., whose cell membrane permeability was increased (0.75 ± 0.11%), whereas the G-CSF group had a reduced frequency of such cells (0.071 ± 0.004%).

### Differentiation of bone marrow cells into cardiac muscle cells:

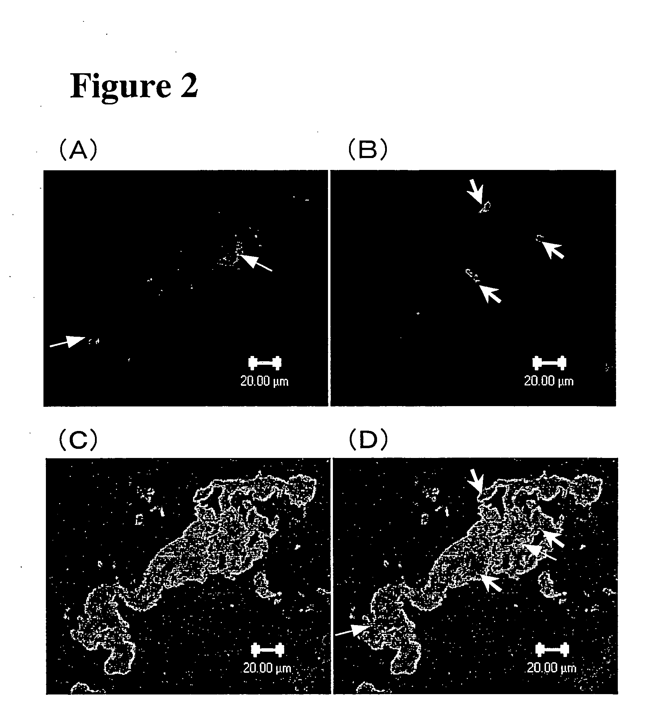

When observed under a confocal laser scanning microscope, DiI-positive and α-sarcomeric actin-positive cells, i.e., bone marrow cell-derived cardiac muscle cells were found in cardiac muscle tissue of the G-CSF group (Figure 2).

### δ-Sarcoglycan:

Expression of δ-sarcoglycan in cardiac muscle tissue was not observed in either group (data not shown).

### (3) Discussion

As demonstrated above, the long-term administration of G-CSF ameliorated progressive myocardial fibrosis, left ventricular remodeling and heart failure in the animal model of cardiomyopathy. The mechanism for such efficacy of G-CSF would be explained by at least the following 4 possibilities:
1) a cardiac muscle-regenerating effect resulting from recruitment of bone marrow cells to cardiac muscle tissue and their differentiation into cardiac muscle cells;
2) an inhibitory effect on loss of cardiac muscle cells;
3) an inhibitory effect on myocardial fibrosis; and
4) an anti-cytokine effect.

1) The possibility of cardiac muscle regeneration by G-CSF was supported by the fact that bone marrow cell-derived cells which were labeled with DiI dye and positive to α-sarcomeric actin (i.e., cardiac muscle cells) were found in cardiac muscle tissue of the G-CSF group when observed under a confocal laser scanning microscope.
2) The ultramicromorphological observation and Evans blue stain suggested the possibility that G-CSF would have an anti-autophagic degeneration/death effect on cardiac muscle cells. The inhibitory effect on loss of cardiac muscle cells (i.e., protective effect on cardiac muscle cells) can be fully deduced from the fact that lesions with loss of cardiac muscle cells are small in the G-CSF group, as indicated by the reduced percent area of left ventricular fibrosis.
3) The anti-fibrosis effect of G-CSF is apparent from Figure 1. As indicated by the increased activity of MMP in cardiac muscle of the G-CSF group, there is an additional possibility that G-CSF will also promote the degradation of collagen fibers through increases in MMP activity, thereby producing the anti-fibrosis effect.
4) Heart failure is known to cause elevated blood levels of cytokines, including tumor necrosis factor α (TNF-α) and interleukin 6 (IL-6). Above all, TNF-α is shown to have an inhibitory effect on cardiac function (Meldrum DR., Am. J. Physiol. 1998, 274: R577-R595). It is therefore estimated that G-CSF will inhibit these cytokines and hence improve cardiac function.

This indicates that G-CSF is effective for human dilated cardiomyopathy.

### INDUSTRIAL APPLICABILITY

The long-term administration of a colony-stimulating factor enables the treatment of non-ischemic heart failure caused by exacerbation of cardiomyopathy.

## Claims

1. A pharmaceutical composition for treating non-ischemic heart failure, which comprises a colony-stimulating factor as an active ingredient.

2. The pharmaceutical composition according to claim 1, wherein the non-ischemic heart failure is caused by exacerbation of cardiomyopathy.

3. The pharmaceutical composition according to claim 2, wherein the cardiomyopathy is idiopathic cardiomyopathy.

4. The pharmaceutical composition according to claim 3, wherein the idiopathic cardiomyopathy is dilated cardiomyopathy.

5. The pharmaceutical composition according to any one of claims 1 to 4, wherein the colony-stimulating factor is granulocyte colony-stimulating factor.

6. A method for treating non-ischemic heart failure, which comprises administering to a patient in need thereof a colony-stimulating factor as an active ingredient in an amount effective for treating non-ischemic heart failure.

7. The use of a colony-stimulating factor for the manufacture of a pharmaceutical composition for treating non-ischemic heart failure.

8. A kit for treating non-ischemic heart failure, which comprises a colony-stimulating factor in an amount effective for treating non-ischemic heart failure, and instructions for use.
